# EUROPEAN PATENT SPECIFICATION

(11) **EP 1 492 630 B1**
(45) Date of publication and mention of the grant of the patent: **17.05.2006**
(21) Application number: 03715089.3
(22) Date of filing: 24.03.2003
(51) Int. Cl.: B05D 3/04, B05D 3/14, C08J 7/18, A61L 15/60

(54) **PREPARATION OF SUPERABSORBENT MATERIALS BY PLASMA MODIFICATION**
HERSTELLUNG VON SUPERABSORBIERENDEM MATERIAL MITTELS PLASMAMODIFIZIERUNG
PREPARATION DE MATERIAUX SUPERABSORBANTS PAR MODIFICATION DU PLASMA

(30) Priority: 23.03.2002 GB 0206932
(43) Date of publication of application: 05.01.2005
(73) Proprietor: Surface Innovations Limited, Wolsingham, County Durham DL13 3HH (GB)
(72) Inventor: BADYAL, Jas, Pal, Singh, Wolsingham, County Durham DL13 3HH (GB); SCHOFIELD, Wayne, Christopher, Edward, Chester CH4 8HH (GB)
(74) Representative: Wood, Graham
(86) International application number: PCT/GB2003/001220
(87) International publication number: WO 2003/080259

(56) References cited:
- EP-A- 0 574 352
- WO-A-02/26871
- WO-A-92/00407
- US-A- 5 236 563
- US-A- 5 318 552
- US-A- 5 972 505

## Description

The invention relates particularly, but not exclusively, to the provision of a method and apparatus for the improvement of plasma processing in the application of coatings to substrates and to the improved control and efficiency in the application of specific coatings.

Plasma modification is widely employed to modify the surface properties of bulk materials and the term is used to describe the use of gases and/or monomers or polymerisation to bring about the modification. By introducing inorganic or organic gases/monomers (including those that are not polymerisable by conventional methods) into the electrical discharge, specific functional groups can be applied onto the substrate. In the case of polymeric substrates, scission of the polymer backbone in the surface region caused by incident ions, photons, and reactive neutrals from the plasma, can often lead to the formation of poorly adherent low molecular weight species. As a consequence, the surface properties can become unstable and disappear over a period of time, or be lost during immersion in a solvent.

The practical and commercial problems caused by this disadvantage are significant as water-absorbing resins are widely used commercially such as in sanitary goods, hygienic goods, wiping cloths, water retaining agents, dehydrating agents, sludge coagulants, disposable towels, and release control agents for various chemicals. Such resins are available in a variety of chemical forms, including substituted and unsubstituted natural and synthetic polymers, such as hydrolysis products of starch acrylonitrile graft polymers, carboxymethyl cellulose, crosslinked polyacrylates, sulphonated polystyrenes, hydrolysed polyacrylomides, polyvinyl alcohols, polyethylene oxides, and many others. These water-absorbing resins are often termed "super absorbent polymers" or SAPs, and typically comprise crosslinked hydrophilic polymers. SAPs are normally capable of absorbing and retaining amounts of aqueous fluids equivalent to many times their own weight, even under moderate pressure. The dramatic swelling and absorbent properties of SAPs are attributed to (a) electrostatic repulsion between the charges along the polymer chains, and (b) osmotic pressure of the counter ions. The ability to absorb aqueous fluids under a confining pressure is an important requirement for a SAP used in a hygienic article, like a diaper.

Conventionally the absorption properties of SAPs are drastically reduced in solutions containing electrolytes, such as saline, urine and blood. The polymers do not function as effective SAPs in the presence of such physiological fluids. The absorption capacity of SAPs for body fluids, like urine and menses, therefore, is dramatically lower than for deionised water because such fluids contain electrolytes. This dramatic decrease in absorption is termed "salt poisoning".

The salt poisoning effect can be explained as follows. Water-absorption and water retention characteristics of SAPs are attributed to the presence of ionisable functional groups in the polymer structure. The ionisable groups typically are carbonyl groups, a high proportion of which are in the salt form when the polymer is dry, and which undergo dissociation and solvation upon contact with water. In the dissociated state, the polymer chains contain a plurality of functional groups having the same electric charge and, thus, repel one another. This electronic repulsion leads to expansion of the polymer structure, which in turn, permits further absorption of water molecules. Polymer expansion, however, is limited by the crosslinks in the polymer structure, which are present in a sufficient number to prevent solubilisation of the polymer. A significant concentration of electrolytes can interfere with the dissociation process of the ionised functional groups, and lead to the "salt poisoning" effect. Dissolved ions such as sodium and chloride ions, therefore, have two effects on SAP gels. The ions screen the polymer charges and eliminate the osmotic imbalance due to the presence of counter ions inside and outside of the gel. The dissolved ions, therefore, effectively convert an ionic gel into a non-ionic gel, and swelling properties are lost.

Numerous investigations are known to have attempted to counteract the salt poisoning effect and therefore improve the absorption performance of SAPs with respect to electrolyte-containing liquids, such as urine and menses. The introduction of both cationic and anionic exchange materials has been and continues to be investigated to alleviate the salt poisoning effect by reducing the salt content in the absorbed liquid. The ion exchanger has no direct effect on the performance of the superabsorbent materials but rather attempts to "condition" the liquid. However, in practise it may not be possible to reduce the salt content sufficiently to have the desired effect on the overall absorption capacity of the combination. In addition, besides being expensive, the ion exchanger has no absorbing effect itself and thus acts as a diluent to the superabsorbent material. It is possible to make anionic superabsorbents with suitable cationic functional groups including quaternary ammonium groups or primary, secondary or tertiary amines that should be present in base form. In fact the most commonly used SAP for absorbing electrolyte-containing liquids, like urine, is neutralised polyacrylic acid. Neutralised polyacrylic acid, however, is susceptible to salt poisoning.

The aim of the present invention is to provide an improved superabsorbent material which allows improved absorbance of liquids in general, and in particular, liquids containing electrolytes.

In a first aspect of the invention there is provided a method of applying a conditioning effect to a material substrate, said method including the step of performing a plasma modification and/or plasma deposition treatment on the substrate, said conditioning effect comprising exposing the substrate to at least two treatment steps: (i) cross-linking of either or both the exterior and internal surfaces of the material; and (ii) plasma modification or plasma deposition of/onto the cross-linked material.

Typically the steps (i) and (ii) are both performed and in sequence onto a superabsorbent material, hereinafter referred to in a non-limiting manner as the substrate.

In one embodiment the precursor gas used in the generation of the plasma is, by way of example only, a noble, inert or nitrogenous gas.

In one embodiment a coating material is modified and in one embodiment is a hydrophilic layer wherein the plasma treatment in the second step acts to oxidise or nitrogenate the same. The precursor gas/liquid used during the second plasma treatment step can be oxygen or nitrogen containing chemical compounds. Any suitable oxidation method can be used, such as ozonolysis

Preferably, when the coating applied is either a hydrophobic/oleophobic layer, the precursor gas/liquid used for the plasma treatment in step (ii) contains fluoride.

Suitable types of plasma and remote plasma can be used and reference to the use of plasma can include the use of any or any combination of pulsed and/or continuous wave plasma and include non-equilibrium plasmas such as those generated by radio frequency (RF), microwaves and/or direct current. The plasma can be operated at low pressures, atmospheric or subatmospheric pressures to suit particular purposes.

In operation, the plasma power applied during the first step can be in the range 0.01 watt to 500 Watts.

In operation the plasma power applied during the second step can be in the range 0.01 watt to 500 Watts.

In one embodiment the plasma power applied during either or both of the first and second steps is pulsed. In addition, or alternatively, the precursor gas/liquid introduced during either or both the first and second steps is pulsed.

Typically the material which is modified is a substrate which is defined as any article which is capable of supporting a coating applied thereto, so it will be appreciated that the same can be rigid or flexible, and can be any of a porous or non-porous substrate such as a film, powder or 3-dimensional article.

In one embodiment, when the substrate is a porous article, the substrate has an exterior surface, a bulk matrix and pores extending from the exterior surface into the bulk matrix, wherein the bulk matrix is, at least in part, polymeric or oligomeric, and the exterior and interstitial surfaces, at least in part, are polymeric or oligomeric.

In one embodiment the porous matrix is a polyolefin. The porous matrix can have a void volume ranging from 0.01% to 99%, but most preferably between 1% and 99%.

In a further embodiment where the substrate is a non-porous article the surface is composed of fabric, metal, glass, ceramic, paper or polymer.

In one embodiment, in step (i) the effect of said step can be controlled to be applied only to a limited depth or throughout the material below the external surface.

In one embodiment, in step (ii) the effect of said step can be controlled to be applied to a limited depth below or throughout the material below the external surface.

In one embodiment the plasma used in either or both steps (i) and (ii) is selectively applied to localised areas across the substrate surface and/or below the substrate surface.

In one embodiment of the invention there is provided an absorbent, hydrophobic polymer, such as polyacrylic acid which is cross-linked by a noble gas plasma, improving its ability to retain water and rendering it super absorbent. Preferably a subsequent nitrogenating plasma then renders said cross-linked polymer compatible with amine functionalities and thus the overall effect of the two step treatment is the formation of a super-absorbent polymer with the ability to retain large quantities of amine containing aqueous solutions.

In one embodiment, products modified in accordance with the invention have application in, for example, the formation of an article for absorbing bodily fluids such as, for example, a nappy (aka diaper), wound dressings, burns treatment, printing techniques, bio integrated circuits, and generally any product where absorbance of liquid is a problematic issue.

Typically, any suitable cross linking method can be used such as e-beam lithography.

The effect of the first step of the method is to improve the thermal stability of the polymer, which in turn means that it can be plasma treated in the second step at higher temperatures. Thus it is preferred that both steps are performed as, without the first step, the second step can cause the polymer to deteriorate.

The invention is also applicable to copolymer and blend coatings thereby having the same advantageous effect.

In addition to improving the characteristic of the substrate surface, the application of material to the substrate surface such as, for example, by plasma deposition, is improved both in the application of the coating and the adhesion of the coating to the substrate surface as the two-step process also leads to an improvement in surface adhesion.

Specific embodiments of the invention are now described with reference to the accompanying diagrams, wherein:-
Structures 1-3 relate to Dimethyl Sulphate, sulphur monoxide and sulphite respectively.
Figures 1A and B illustrate the water contact angle of polypropylene film exposed to varying levels of argon plasma crosslinking followed by plasma polymerisation of dimethyl sulphate; (a) before washing; and (b) after washing with propan-2-ol;
Figures 2a and b illustrate the water absorption profiles for plasma polymerisation of dimethyl sulphate onto a porous nonwoven polypropylene stack as a function of power: (a) before washing; and (b) after washing with propan-2-ol;
Figures 3a and b illustrate the water absorption capacity of the outermost layer of porous non-woven polypropylene stack as a function of argon plasma and dimethyl sulphate plasma power level settings: (a) before washing; and (b) after washing with propan-2-ol;
Figures 4a and b illustrates the XPS spectra following dimethyl sulphate plasma polymerisation onto non-porous polypropylene film as a function of input power: (a) S(2p); and (b) C(1s);
Figure 5 illustrates the XPS S/C ratios at various power level settings for dimethyl sulphate plasma polymerisation (in the absence of argon plasma pre-treatment);
Figures 6a and b illustrate the XPS S/C ratios of dimethyl sulphate plasma polymers deposited onto a porous non-woven polypropylene stack at 3 W and 10 W before and after propan-2-of washing: (a) no pre-treatment; and (b) 50 W Ar plasma pre-treatment;
Figure 7 illustrates the Substrate subtracted ATR FTIR spectra of dimethyl sulphate plasma polymer deposited onto non-porous polypropylene film as a function of power level;
Figures 8a-e illustrate the optical microscopy images of porous non-woven polypropylene fibres; (a) untreated; (b) dimethyl sulphate (3 W); (c) dimethyl sulphate (10 W); (d) argon (50 W) and dimethyl sulphate (3W); and (e) argon (50 W) and dimethyl sulphate (10 W);
Figure 9 illustrates absorption under load of test liquids by treated polyacrylic acid SAPs modified at various argon plasma power levels;
Figure 10 illustrates absorption under load of test liquids by treated polyacrylic acid SAPs modified at various nitrogen plasma power levels;
Figure 11 illustrates absorption under load of test liquids by treated polyacrylic acid SAPs modified by argon plasma at 20 W and various nitrogen power levels;
Figure 12 illustrates substrate subtracted ATR FTIR spectra of polyacrylic acid modified with nitrogen plasma at (a) untreated polyacrylic acid; (b) 5 W; (c) 10 W, and (d) 20 W;
Figures 13a and b illustrate water contact angles of polypropylene films exposed to varying levels of argon plasma crosslinking followed by air plasma treatment;
Figure 14 illustrates water absorption profiles for air plasma treated porous non-woven polypropylene stack at various powers;
Figures 15a and b illustrate top layer water absorption of non-woven polypropylene stack;
Figure 16 illustrates XPS O/C rates of polypropylene films as a function of air plasma power input; and
Figure 17a-f illustrate optical micrographs of non-woven porous polypropylene.

In this first specific, illustrative embodiment, relatively small (6 cm x 2 cm) strips of non-porous polypropylene film (capacitor grade, ICI, 0.5 *µ*m thickness) and also porous non-woven polypropylene film (Corovin GmbH, MD300A, 125 *µ*m thickness) were rinsed in non-polar (cyclohexane) followed by polar (propan-2-ol) solvents, and then dried in air. In the case of the porous substrate, 8 sheet stacks were used in order to evaluate the depth of plasma penetration.

Plasma crosslinking and deposition treatments were carried out in a cylindrical glass reactor pumped by a rotary pump via a liquid nitrogen cold trap (base pressure = 1 x10⁻² mbar, leak rate = 9.9 x 10⁻⁹mol s⁻¹). A copper coil wrapped around the reactor was connected to a 13.56 MHz radio frequency power supply via an LC matching network. Prior to each experiment the chamber was cleaned using an air plasma operating at 50 W and 0.2 mbar. At this stage the polymer substrate was placed into the centre of the reactor. The noble gas pre-treatment step entailed introducing Argon (99% purity, Air Products) at a pressure of 0.2 mbar followed by plasma ignition for 5 min. Immediately afterwards dimethyl sulphate precursor (Aldrich, 99% purity, further purified using several freeze-pump-thaw cycles) was introduced via a fine control needle valve at a pressure of 0.1 mbar and 4.0 x 10⁻⁸ mol s⁻¹ flow rate followed by re-ignition of the electrical discharge for 5 min.

Film thickness measurements were carried out using an nkd-6000 spectrophotometer (Aquila Instruments Ltd). Transmission-reflectance curves (350-1000 nm wavelength range) were fitted to a Cauchy model for dielectric materials using a modified Levenburg-Marquardt method.

For the non-porous polypropylene film substrates, sessile drop water contact angle measurements were carried out using a video capture apparatus (AST Products Inc., Model VCA 2500). Each contact angle value was acquired 10 s after dispensing a 2 *µ*l drop of high purity water onto the surface. In the case of the porous non-woven polypropylene film substrate, water absorption measurements were adopted as a means for following changes in wettability. This entailed immersion of individual plasma treated sheets into 1 ml of aqueous dye solution (0.625 wt % solution of blue dye Coumarin 47, Parker Pen Company). Any remaining excess liquid was then combined with water to make a 1 ml aliquot and analysed by UV-VIS absorption spectroscopy at 200 nm (this wavelength corresponds to dye absorption) using a UNICAM UV4 spectrophotometer. Reference was made to a set of calibration solutions. The possibility of there also being low molecular weight species present on the plasma treated surfaces was investigated by rinsing the samples in propan-2-ol,and then re-examining their surface wettability.

A VG Escalab spectrometer equipped with an unmonochromatised Mg K_{*a*} X-ray source (1253.6 eV) and a concentric hemispherical analyser was used for X-ray photoelectron spectroscopy (XPS) analysis of the modified surfaces. Elemental compositions were calculated using sensitivity factors derived from chemical standards, O(1s): C(1s): S(2p) equals 0.45: 1.00: 0.60.

Substrate subtracted attenuated total reflectance (ATR) infrared spectra of plasma polymer films deposited onto polypropylene film were acquired using a diamond ATR accessory (Graseby Specac Golden Gate) fitted to a Perkin Elmer Spectrum One FTIR spectrometer. Spectra were acquired at a resolution of 4 cm⁻¹ over 500-4000 cm⁻¹ wavelength range using a liquid nitrogen cooled MCT detector.

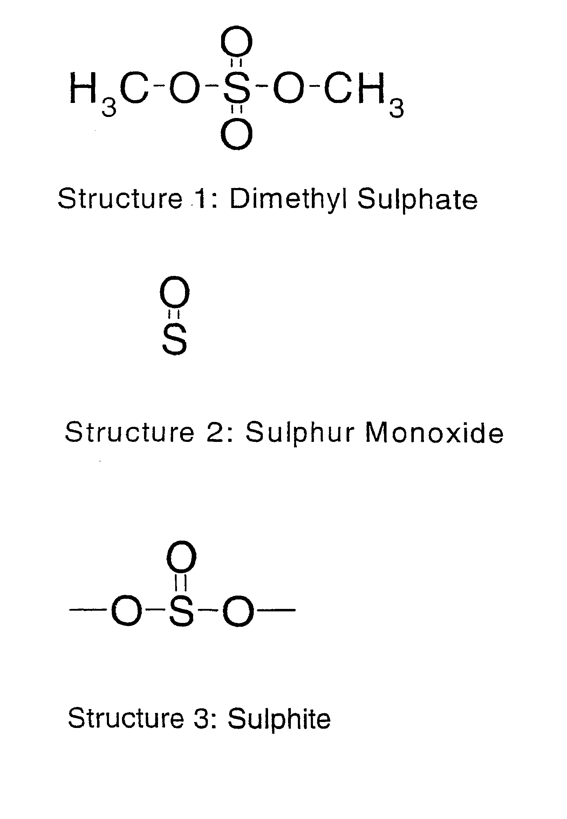

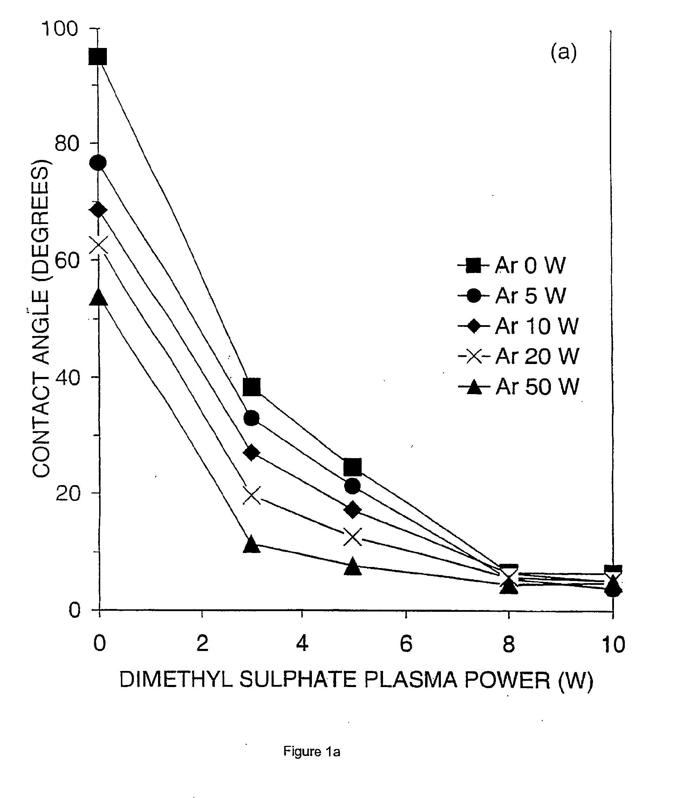

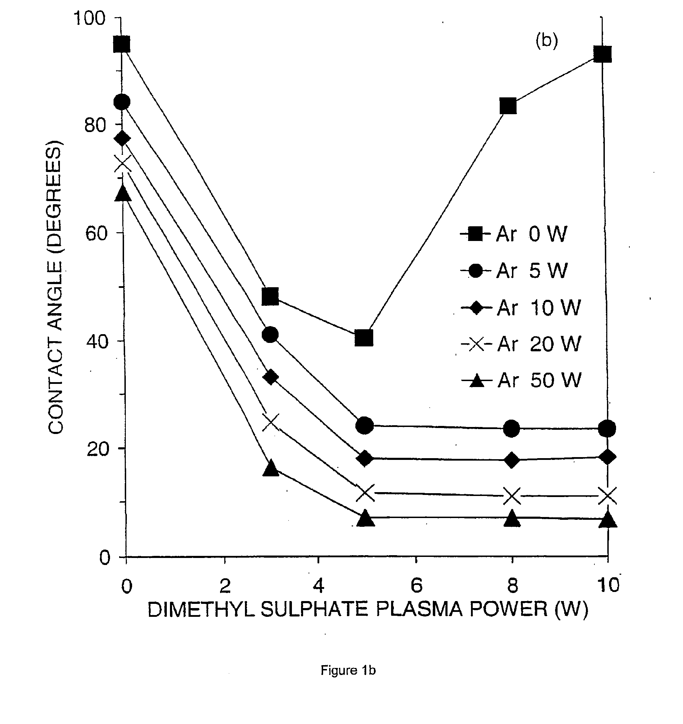

In analysis of the results a water contact angle value of 95° ± 3 was measured for the untreated non-porous polypropylene film surface. Plasma polymerisation of dimethyl sulphate directly onto this substrate produced an improvement in hydrophilicity consistent with previous studies, as shown in Figure 1A. A decrease in contact angle value was observed with rising power, eventually reaching a limiting value of around 6° around 8 W. Higher power settings produced no further improvement in surface wettability. Washing these plasma modified surfaces with propan-2-ol indicated a divergence in performance, as shown in Figure 1B. Retention of hydrophilicity was greatest at lower power level settings, whereas at higher powers, the water contact angle value became more reminiscent of the original untreated polypropylene substrate (i.e the deposited plasma polymer layer was being dislodged from the surface by solvent).

Argon plasma pre-treatment prior to plasma polymerisation of dimethyl sulphate produced two beneficial effects. Firstly, an improvement in surface wettability was noted, and in addition, the deposited dimethyl sulphate plasma polymer layer exhibited greater stability towards solvent removal, Figure 1B. The most hydrophilic and stable surfaces were achieved by using a combination of high power levels for both argon plasma crosslinking and plasma polymerisation of dimethyl sulphate.

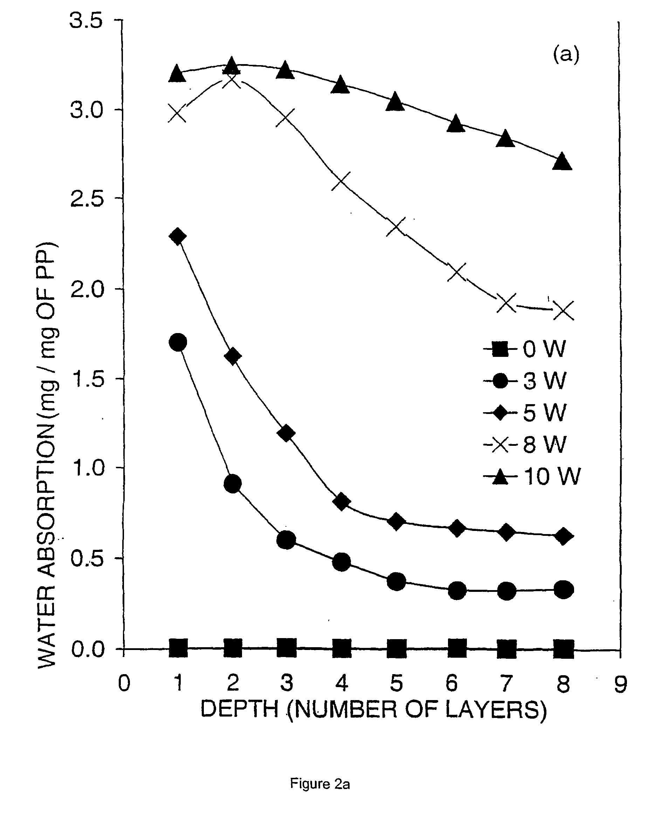

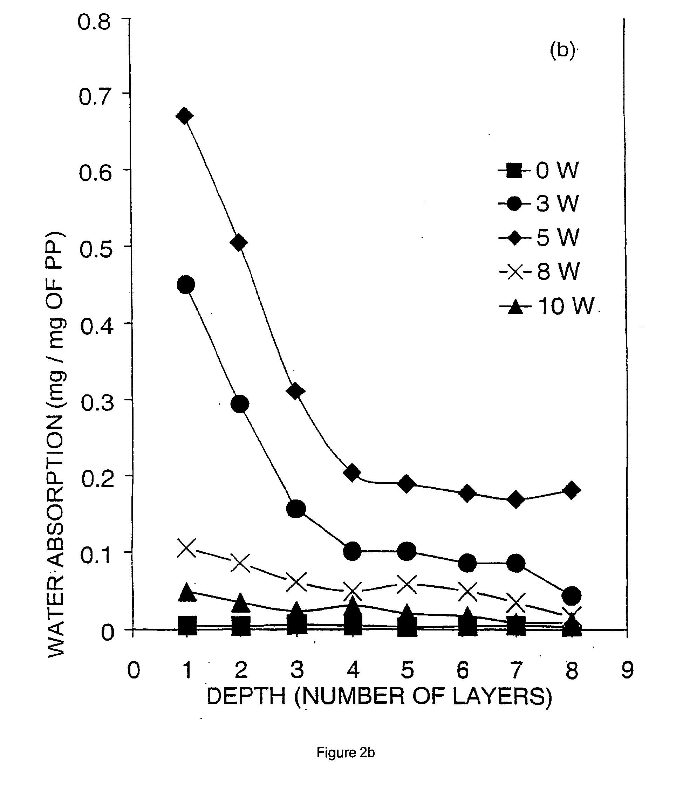

Water absorption measurements for dimethyl sulphate plasma polymer layers deposited onto porous non-woven polypropylene films displayed several important attributes. Firstly, the degree of hydrophilicity was dependent upon depth, Figure 2a. Also, the level and penetration of water uptake improved at higher power settings. However all of these plasma polymerised dimethyl sulphate layers were found to be unstable towards solvent washing, Figure 2b. This behaviour is analogous to the poor hydrophilicity observed for dimethyl sulphate plasma polymer deposited onto non-porous polypropylene films, Figure 1b.

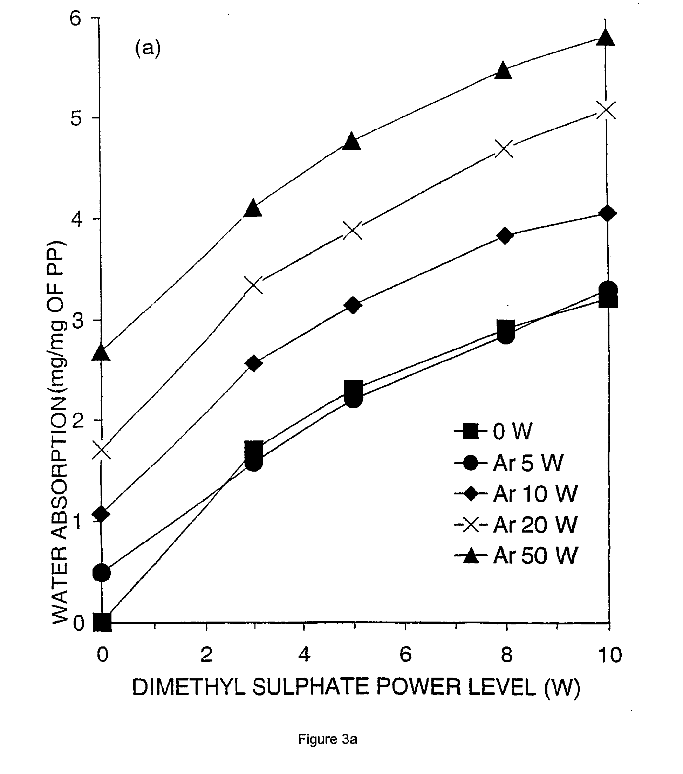

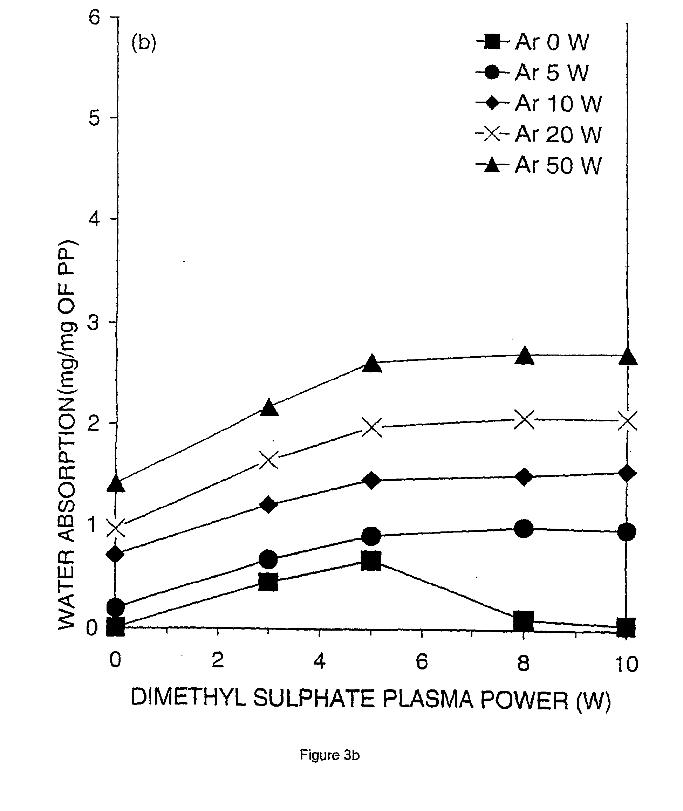

Argon plasma crosslinking pre-treatment of the porous non-woven polypropylene films prior to plasma polymerisation of dimethyl sulphate gave rise to a significant improvement in water absorption capacity (up to 600% water uptake by weight), also a corresponding improvement in stability towards propan-2-ol washing was evident, Figures 3a and b. The degree of enhancement critically depends upon the power level settings for both plasma treatment steps. In the case of solvent washing, argon plasma power level was found to be the governing factor at powers greater than 5 W. It is of interest to note that a certain degree of water absorption occurs for just dimethyl sulphate vapour exposure to the argon plasma activated polypropylene surface (whereas there is no water absorption following dimethyl sulphate exposure to unactivated polypropylene).

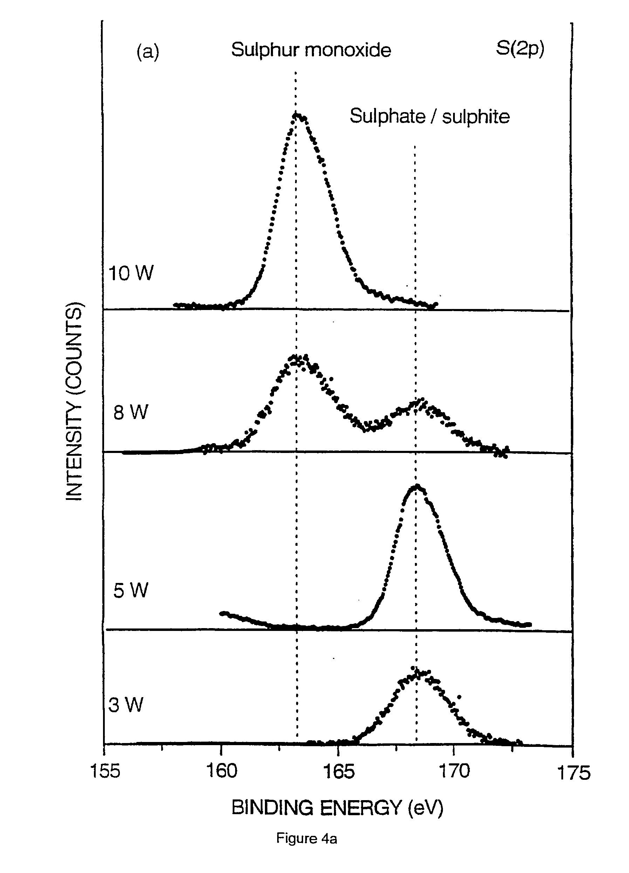

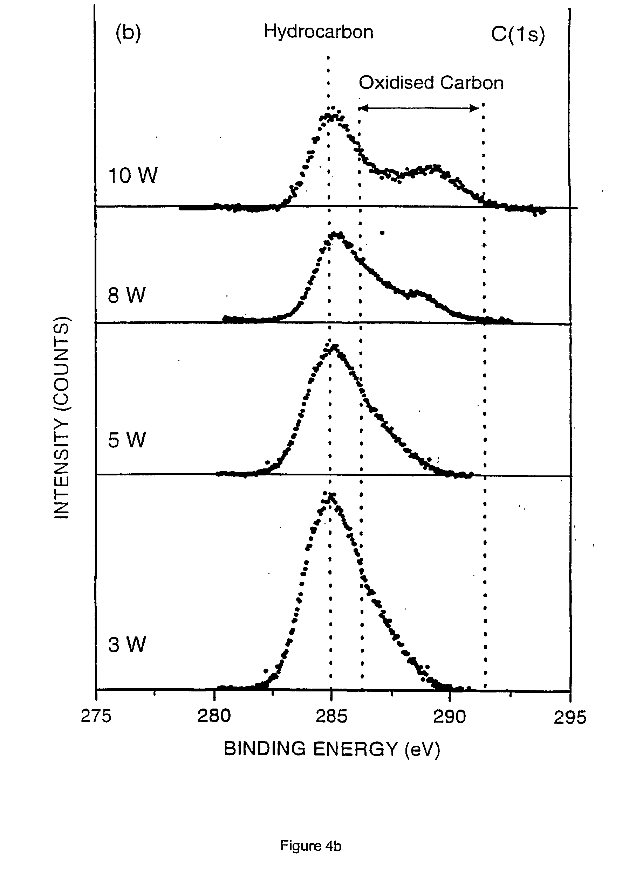

For the deposited dimethyl sulphate plasma polymer layers, XPS analysis indicated a strong correlation between the concentration of high oxidation state sulphur species and surface hydrophilicity, as shown in Table 1 and Figure 4.

**Table 1: XPS elemental analysis of dimethyl sulphate plasma polymer deposited onto polypropylene film.**

| | | | |
|---|---|---|---|
| **Dimethyl sulphate power level (W)** | **%C** | **%O** | **%S** |
| 3 | 60 ± 0.5 | 29 ± 0.5 | 8 ± 0.5 |
| 5 | 31 ± 1.0 | 55 ± 0.6 | 14 ± 0.5 |
| 8 | 23 ± 0.6 | 60 ± 0.6 | 17 ± 0.5 |
| 10 | 19 ± 0.5 | 63 ± 0.6 | 18 ± 0.5 |
| Theoretical | 29 | 57 | 14 |

The lower binding energy S(2p) peak at 164.8 eV can be assigned to sulphur atoms bonded to one or two oxygen atoms (e.g. sulphur monoxide groups (Structure 2), whilst the higher binding energy component at 169.4 eV is typical of sulphate (Structure 1) and sulphite (Structure 3) environments. The shift towards less oxidised sulphur centres at higher plasma powers was compensated by the emergence of a larger proportion of oxidised carbon species in the C(1s) spectra, Figure 4.

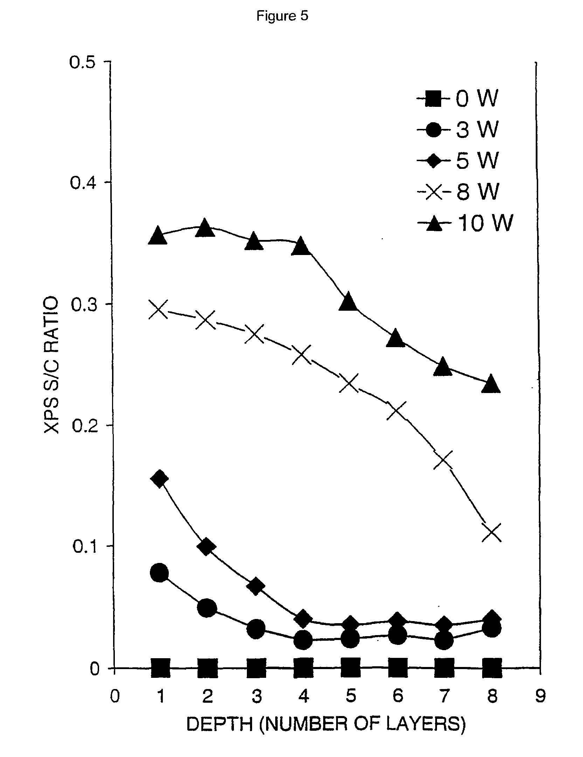

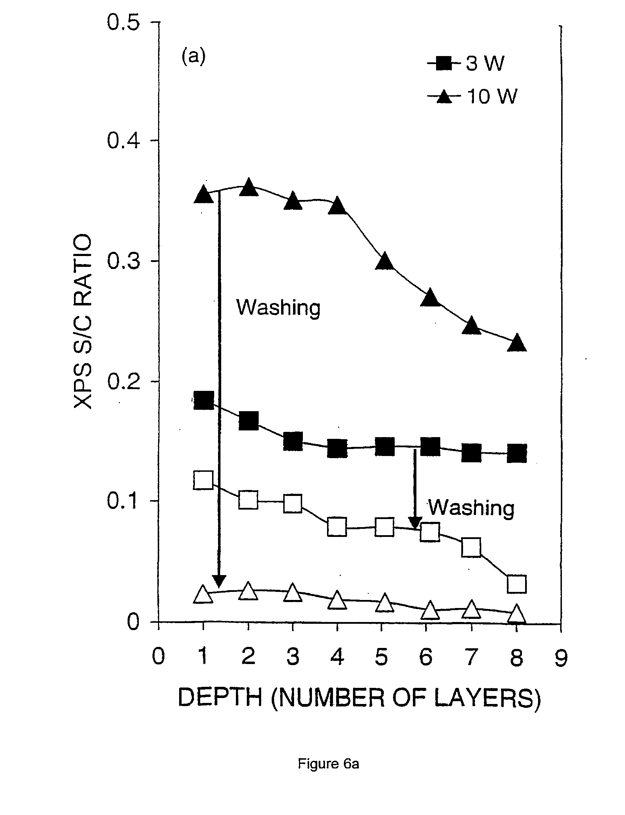

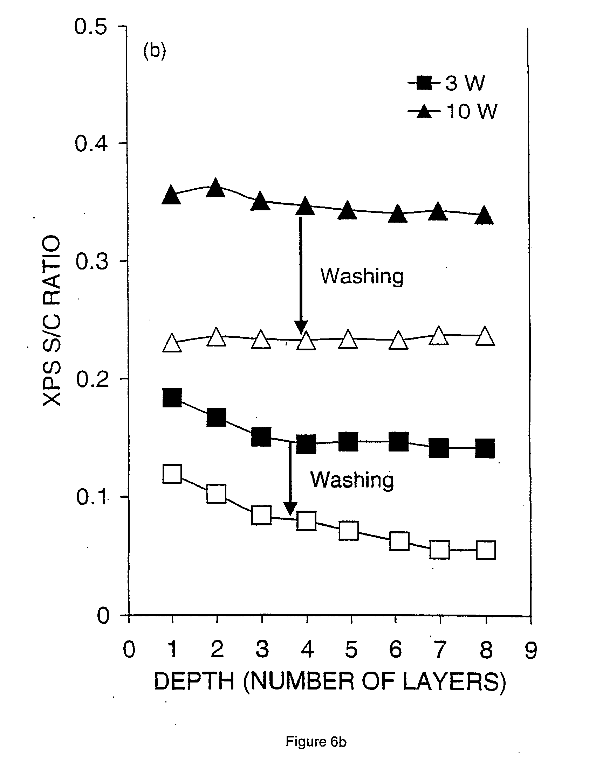

In the case of the porous polypropylene film, stacks XPS verified that the extent of plasma penetration was important, Figure 5. Poor penetration occurred at low plasma power settings. Deeper modification was observed for higher plasma power levels, however this was accompanied by poor stability towards solvent washing, Figure 6 (as seen previously in Figures 1a and b and 3a and b). Argon plasma pre-treatment was found to improve the level of sulphur incorporation and stability towards solvent washing, as shown in Figure 6 and this is consistent with the observed improvement in water absorption properties, as shown in Figures 3a and b.

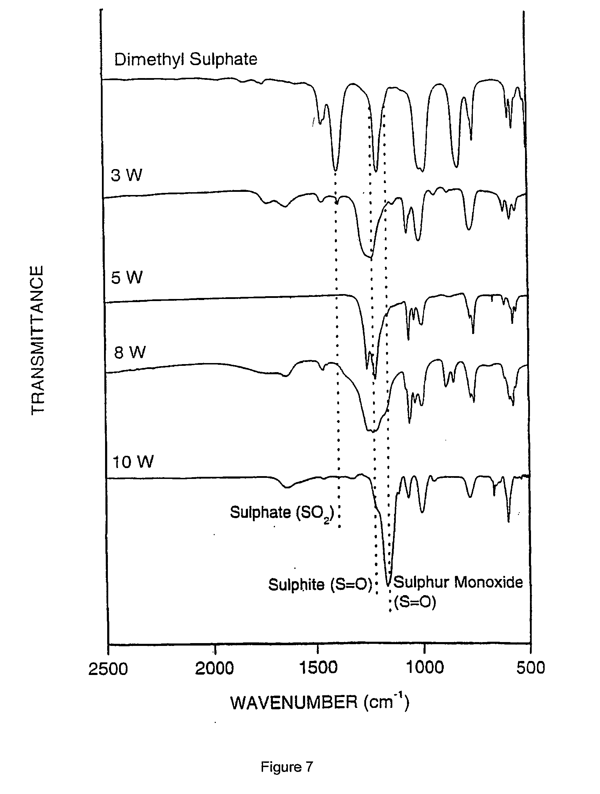

Infrared spectroscopy of the dimethyl sulphate monomer gave the following major band assignments: O=S=O antisymmetric stretching (1456 and 1391 cm⁻¹), O=S=O symmetric stretching (1199 cm⁻¹), S-O stretching (1004 and 983 cm⁻¹) and O-S-O stretching (at 826 and 758 cm⁻¹), Figure 7. Dimethyl sulphate plasma polymer layers deposited at both 3 W and 5 W settings gave rise to a prominent unsaturated sulphur bond feature corresponding to sulphite S=O stretching (ca 1225 cm⁻¹), where the splitting (in particular 5 W) may be due to rotational isomerism around sulphite S-O single bonds (Structure 3), i.e. the sulphate stretches characteristic of the monomer have disappeared to be replaced by sulphite groups. At higher power levels, (8 W and 10 W), a new sulphur monoxide band appeared at 1168 cm⁻¹. The presence of a mixture of both sulphite and sulphur monoxide bands at 8 W correlates well with the doublet seen in the S(2p) XPS data at this power setting, Figure 4.

Reflectometer measurements provided values of increasing deposition rates for thin films of the plasma polymers with rising power level settings, Table 2.

**Table 2: Reflectometer thickness measurements of dimethyl sulphate plasma polymer layers deposited onto polypropylene film.**

| | |
|---|---|
| **Plasma power level used (W)** | **Deposition rate (nm/min)** |
| 3 | 17 ± 3.2 |
| 5 | 26 ± 2.4 |
| 8 | 33 ± 2.1 |
| 10 | 39 ± 1.8 |

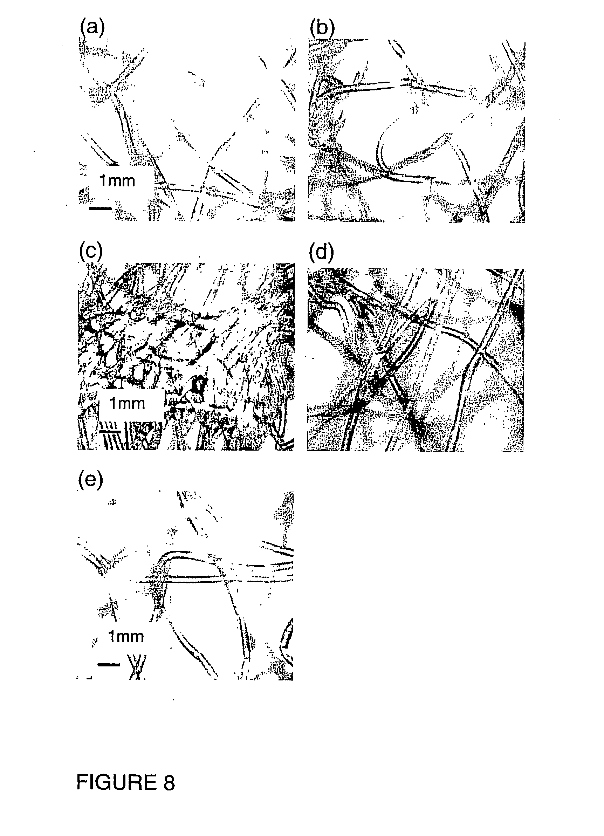

Optical microscopy showed considerable agglomeration of non-woven polypropylene fibres during the direct plasma polymerisation of dimethyl sulphate at power levels above 5 W, Figure 8. Argon plasma cross-linking pre-treatment was found to alleviate this drawback and provided good structural retention of the hydrophilic fibres.

It is therefore clear from these results that plasma polymerisation of dimethyl sulphate leads to an improvement of surface wettability due to the incorporation of hydrophilic sulfur-containing groups. Low power levels give better structural retention of sulphite groups, Figures 4 and 8. Whilst higher power settings lead to more extensive monomer fragmentation culminating in the incorporation of sulphur monoxide centres. The greater vulnerability of the deposited plasma polymer to solvent removal at high electrical discharge powers can be attributed to the creation and etching properties of atomic oxygen, this can lead to the formation of low molecular weight (loose) material on the surface.

Noble gas plasma pre-treatment was found to significantly improve the wettability and adhesion of deposited dimethyl sulphate plasma polymer films. This can be explained in terms of crosslinking, and the formation of trapped free radicals at the polyolefin surface. A crosslinked polypropylene surface will be less susceptible towards chain sissions and the formation of low molecular weight material. Whilst the entrapped free radicals at the surface can participate in chemical bonding interactions during subsequent exposure to dimethyl sulphate plasma species.

In the case of porous non-woven polypropylene substrates, plasma penetration of both argon and dimethyl sulphate plasmas improved at higher power settings, Figures 2 and 3. Under such conditions, more energetic species are generated (greater plasma sheath potential), which are capable of penetrating deeper into the subsurface. Argon plasma pre-treatment (surface crosslinking) in combination with plasma polymerisation of dimethyl sulphate at higher powers (necessary for penetration into the sub-surface) was found to produce the most stable hydrophilic surfaces. This can be attributed to noble gas plasma crosslinking raising the melting temperature of the polypropylene fibre surface, and thereby helping to retain its structural integrity, Figure 8. Such hydrophilic surfaces have potential use in filters, adhesion promotion and biocompatibility.

In a further illustrative example of the application of this invention, polyacrylic acid granules (partially neutralised and slightly crosslinked (5%), ASAP 2000, Chemdal International Ltd) were placed onto a glass plate (50 mm x 15 mm) and placed into a cylindrical glass reactor pumped by mechanical rotary pump via a liquid nitrogen cold trap (base pressure = 9 x 10⁻³ Torr, an leak rate = 4.1 x 10⁻⁹ mol s⁻¹). A copper coil wrapped around the reactor was coupled to a 13.56 MHz radio frequency power supply via an LC matching network. Prior to each experiment, the chamber was cleaned using a 50 W air plasma at 26.7 Pa (0.2 Torr). Samples were exposed to a source gas, introduced via a fine control needle valve at a pressure of 26.7 Pa (0.2 Torr), followed by plasma exposure for 5 minutes. Upon completion, the reactor was purged with monomer for 5 minutes.

Absorption under load (AUL) is a measure of the ability of an SAP to absorb fluid under an applied pressure. The AUL was determined by the following method, as described elsewhere. A sap (0.100 g ± 0.001 g) was carefully scattered onto a 140-micron, water permeable mesh attached to the base of a hollow plexiglass cylinder with an internal diameter of 25 mm. The sample was covered with a 100 g cover plate and the cylinder assembly weighed. This gives an applied pressure of 20 g.cm⁻² (0.28 psi). The screened base of the cylinder was placed in a 100 mm petridish containing 25 ml of a test solution, and the polymer was allowed to absorb for 1 hour. By reweighing the cylinder assembly, absorption under load (AUL) at the given pressure was calculated by dividing the weight of the liquid absorbed by the dry weight of the polymer before liquid contact.
Test solutions used were distilled water, 0.9% saline solution and a 0.9% ammonia solution both to show the effects of salt poisoning and the uptake of materials such as urine, blood etc.

ATR-FTIR was used to probe the plasma treated polymer granules on a Perkin Elmer Spectrum One FTIR instrument at a resolution of 4 cm⁻¹ and averaged over 64 scans between 4000 - 700 cm⁻¹ using a liquid nitrogen cooled MCT detector.

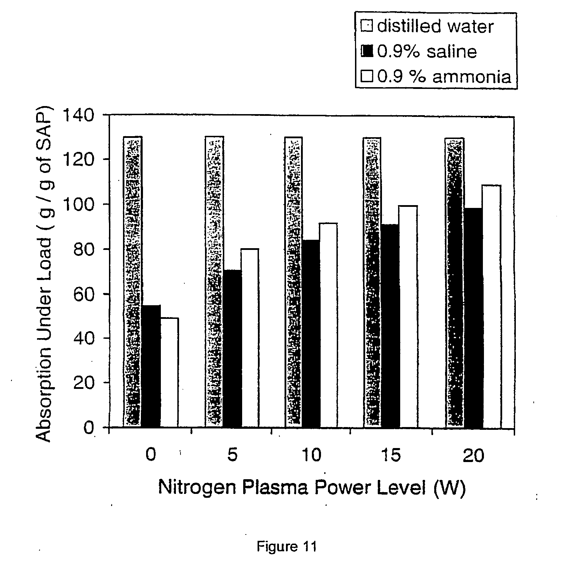

Poor absorbency of the lightly crosslinked partially neutralised polyacrylic acid untreated material towards electrolyte containing liquids was found by comparing deionised water with saline and ammonia solutions, Figure 11. 105.6 g of deionised water was found to absorb per gram of polymer at 20 g.cm⁻² (0.28 psi). In contrast, the same material was only capable of absorbing 29.7 g, and 23.5 g of the saline and ammonia solutions at 20 g.cm⁻² (0.28 psi), respectively. This dramatic decrease in absorption is attributed to the salt poisoning effect.

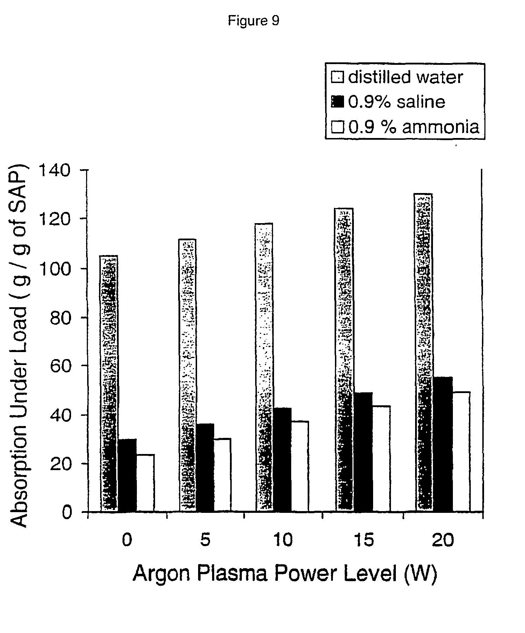

Argon plasma treatment of the polyacrylic acid produced an increase in absorptive capacity, Figure 9. However, the individual levels of absorption for both the saline and ammonia solutions remained significantly lower than corresponding distilled water absorption levels (i.e. the salt poisoning effect was still present).

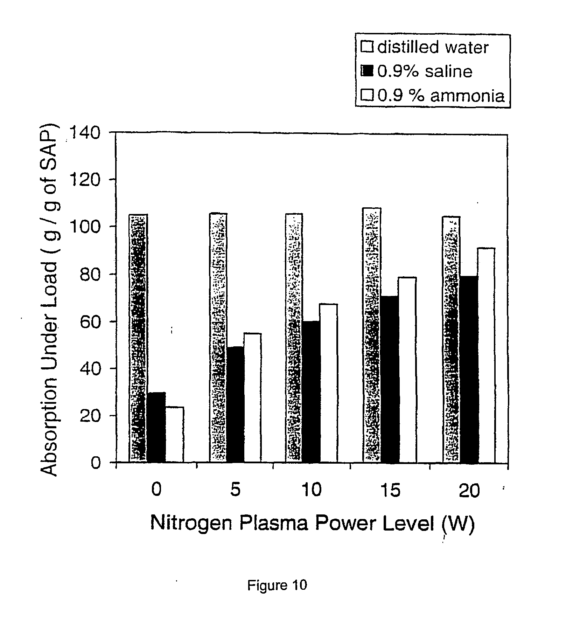

In the case of nitrogen plasma treatments, the absorption properties as measured by AUL were divergent, Figure 10. The absorption capacity of distilled water remained unaffected by the action of nitrogen plasma. However, substantial increases in performance of the absorptive capacity of polyacrylic acid with respect to both saline and ammonia solutions were found, Figure 11. The maximum absorptions (96 g / g and 79 g / g for ammonia and saline solutions respectively) are comparable to the performance obtained using distilled water indicating that the salt poisoning effect had been overcome. It is of relative interest to note that the rate of ammonia absorption improves to that of saline solution under the action of nitrogen plasma.

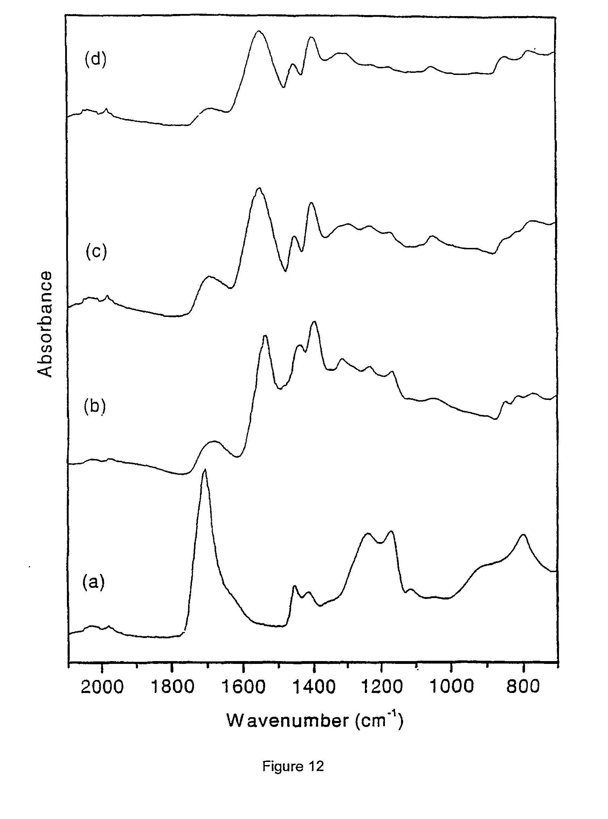

A combination of argon plasma pre-treatment at 20 W for 5 minutes, followed by nitrogen plasma treatment indicated further improvements of the absorptive capacity of the SAP materials, Figure 12. The effect of overcoming the salt poisoning effect and enhancing the absorption due to crosslinking generates materials that can be termed and used as SAPs.

The absorption measurements of SAPs that overcome the salt poisoning effect by modification using a nitrogen plasma is supported by infrared data, Figure 12. The emergence of antisymmetric and symmetric carbonyl salt peaks at 1540 cm⁻¹ and 1410 cm⁻¹ respectively for nitrogen plasma treated polyacrylic acid is contrasted with the substantially reduced carbonyl stretching for the untreated polyacrylic acid at 1706 cm⁻¹. The generation of salt peaks under the action of nitrogen plasma is attributable to the incorporation of ammonium counter ions coordinated around the carboxylic acid centres, effectively generating a neutralised salt of polyacrylic acid with a basic form.

The production of SAPs that overcome the salt poisoning effect and can have enhanced absorptive capacity under pressure is possible without the use of conventional chemical additives such as ion exchangers or surfaces crosslinkers which dilute the properties of the SAPs. Plasma modification of polyacrylic acids using argon and/or nitrogen gas can generate SAPs that substantially reduce the salt poisoning effect even under pressure.

In a yet further utilisation of the invention small (6cm x 2 cm) strips of non-porous (capacitor grade, ICI, 0.5 µm thickness, Corovin GmbH) polypropylene film were cleaned in non-polar (cyclohexane) and polar (propane-2-ol) solvents and then dried in air. For the porous material, 8 sheets were stacked to give an overall thickness of 1 mm in order to investigate the extent of plasma penetration.

Radio frequency (RF) power at 13.56 MHz was applied through a copper cil wound around the outside of a tubular glass reactor (3 dm³ volume capacity, with an inner diameter of 5 cm and a length of 68 cm). A Faraday cage was placed around the apparatus to prevent the leakage of stray electromagnetic radiation. A typical experimental run comprised evacuating the plasma chamber to a base pressure of 2 x 10⁻³ mbar using a liquid nitrogen trapped mechanical rotary pump. Feed gas was then introduced at 0.2 mbar pressure and the electrical discharge ignited. Plasma exposure time was kept constant at 5 mins in all cases. Both the influence of argon plasma pre-treatment, and subsequent air plasma exposure were investigated in terms of power settings.

Sessile drop water contact angle measurements were carried ot on the non-porous polypropylene films using a video capture apparatus (AST Products Inc. VC2500). Each contact angle value was taken for a 2 *µ*l drop of high purity water syringed onto the surface.

In the case of the porous polypropylene film substrate bulk water absorption was used to determine the change in wettability. A 0.625% wt blue dye aqueous solution of Coumarin 47 (Parker Pen Company) was used as the probe liquid. UV absorption measurements were taken for each layer in the stack using a UV-VIS Spectrophotometer UNICAM UV4. This comprised immersion of the plasma treated sheet into 1 ml of the aqueous dye solution. Any remaining excess liquid was then combined with water to make a 1 ml aliquot, and this was analysed by UV-VIS absorption spectroscopy at 200 nm (this wavelength corresponds to dye absorption). By referencing to a set of known concentration calibration solutions, it was possible to determine the water absorption capacity of the plasma treated porous films as a percentage of the polymer mass prior to soaking.

The stability of the plasma modified samples towards hydrophobic recovery was evaluated by rinsing them in propan-2-ol in order to remove any low molecular weight oxidised material generated at the surface, followed by re-evaluation of surface wettability.

A VG ESCALAB spectrometer equipped with an unmonochromatised Mg Kₐ X-ray source (1253.6 eV), and a concentric hemispherical analyser was used for surface analysis by X-ray photoelectron spectroscopy (XPS). Elemental sensitivity (multiplication) factors were taken as being O(1s): C(1s) equals 0.45: 1.00.

In the case of the non-woven porous polypropylene films, the morphological nature of the constituent fibres was examined by optical microscopy. An Olympus BX40 optical microscope equipped with a digital camera and a fibre optic light source (Euromax) was used for image capture.

A series of results were analysed as is now described.

### (a) Flat Polypropylene Film

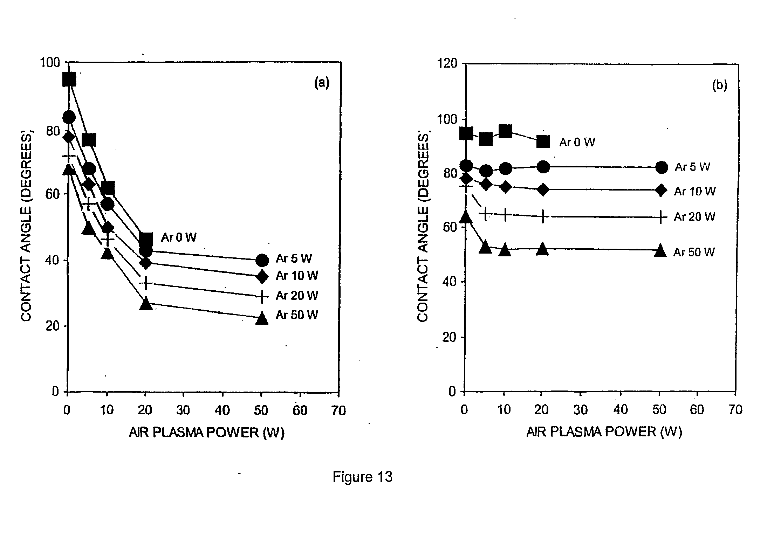

A water contact angle value of 95 ± 3° was measured for the untreated non-porous polypropylene film surface. In the case of straightforward air plasma treatment, the contact angle was found to decrease with increasing power, to eventually reach a minimum of 45° at 20 W (higher power levels caused physical damage to the samples) as shown in Figure 13a. Washing the plasma modified polymer surfaces in propan-2-ol gave rise to the loss of hydrophilicity, with the water contact angle value becoming reminiscent of that associated with untreated polypropylene film as shown in Figure 13b.

In the case of argon plasma, pre-treatment followed by air plasma oxidation, provided two beneficial effects. Firstly, an improvement in surface wettablity, Figure 13a and b. Also, argon plasma crosslinking improved the stability of the oxidised surface towards solvent washing (hydrophobic recovery). Control experiments comprising just argon plasma exposure were found to be not as effective (in this case free radical sites at the plasma modified surface undergoing oxidation upon exposure to air); however these oxidised surfaces were also stable towards hydrophobic recovery. The extent of argon plasma crosslinking (i.e argon plasma power level) was found to govern the stability towards hydrophobic recovery following air plasma exposure.

### (b) Porous Polypropylene Sheet

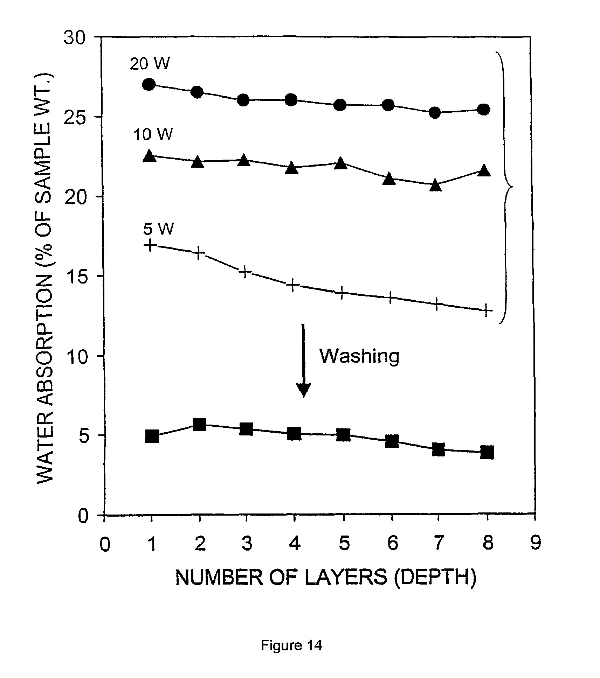

The stabilising influence of argon plasma pre-treatment upon the hydrophilicity of air plasma modified polypropylene surfaces was further investigated by examining porous non-woven substrates. Dye absorption measurements showed that the poor retention of hydrophilicity previously seen for the flat films following air plasma treatment and solvent washing was also evident for these porous substrates, Figure 14. It is of interest to note that for just air plasma exposure, hydrophilic incorporation occurred in a fairly uniform manner with depth down to 8 layers (1 mm), Figure 14. This data is consistent with a large penetration depth of plasma species into the porous substrate. A slight reduction (from 17% to 13%) was observed at very low powers (5 W) where the density of energetic particles becomes a limiting factor. Propan-2-ol solvent washing of the modified substrate produced a significant drop in water absorption to approximately 5% (this is close to the untreated value of 0%). This behaviour is analogous to the corresponding investigation undertaken with the flat non-porous polypropylene films.

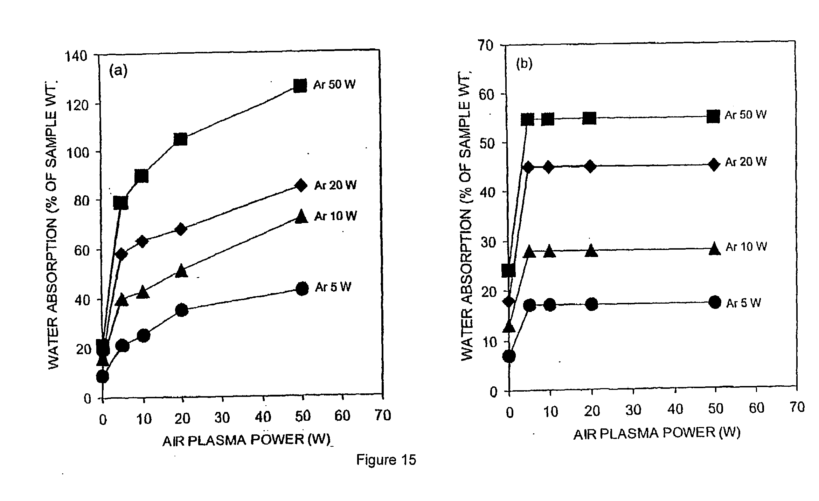

In the case of argon plasma, crosslinking prior to air plasma treatment, a significant improvement in water absorption was found both prior to and following propan-2-ol washing, as shown in Figures 15a and b. In the former case, there is a good correlation between both air and argon plasma power level and the amount of water absorption. Whilst only the power input for argon plasma treatment appeared to influence retention of hydrophilicity subsequent to propan-2-ol washing. Control experiments using just argon plasma treatment (i.e Air power level = 0 W) exhibited much lower levels of surface modification with stability towards hydrophobic recovery during solvent rinsing.

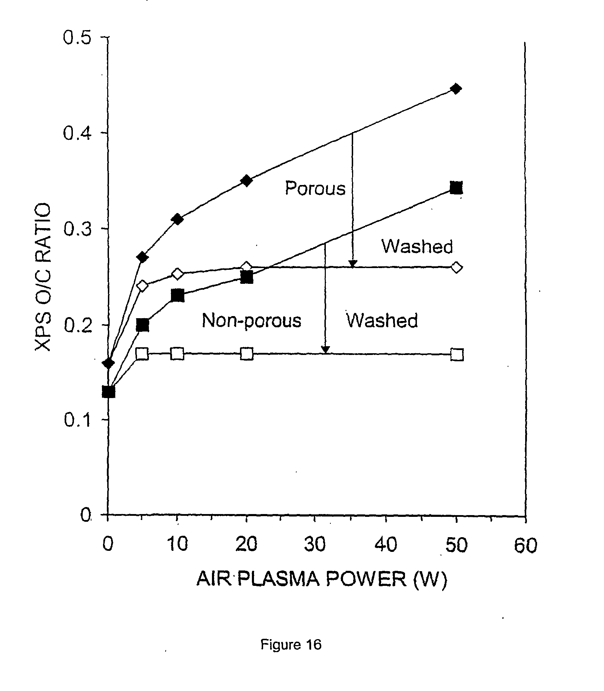

XPS analysis showed that a good correlation exists between surface hydrophilicity and O/C atomic ratios, as shown in Figure 16. A significant improvement in O/C ratio was noted for the two-step plasma treatment, thereby confirming the importance of argon plasma crosslinking.

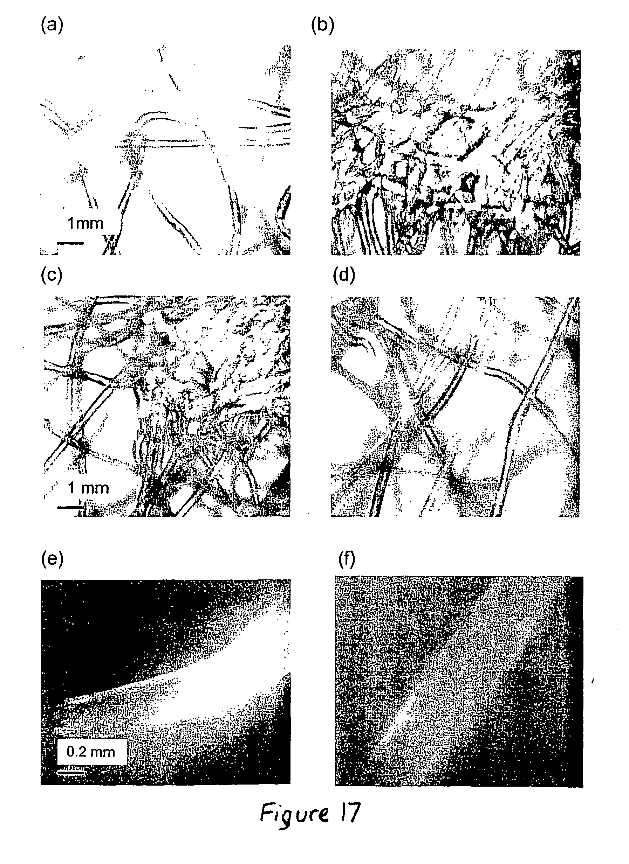

Optical microscopy of the untreated polypropylene non-woven film showed the presence of randomly orientated fibres, as illustrated in Figure 17. In the case of just air plasma treatment, the fibre surface becomes etched and parts of the film appear highly densified due to localised melting. The extent of damage was found to correspond to the air plasma power level setting. Argon plasma crosslinking pre-treatment alleviated deterioration of the substrate. In this case, the fibres retained the structure previously identified for the untreated non-woven surface.

For just air plasma modification, the surface wettability of polypropylene can be unstable and easily removed by solvent washing. This can be attributed to the formation of a layer of oxidised low molecular weight material on the surface generation by polymer chain scission.

Noble gas plasma treatment of polymeric materials comprises interactions of energetic particles and electromagnetic radiation with the surface. Some of the photons possess sufficient energy to break chemical bonds in the surface region and create radicals, which subsequently undergo cross-linking. Any trapped radicals become oxidised upon exposure to air leading to an improvement in surface wettability with low hydrophobic recovery. This can be ascribed to a partially oxidised and crosslinked surface layer.

Argon plasma exposure prior to air plasma treatment imparts two major benefits on surface hydrophilicity. Firstly, there is an enhancement in water contact angle and absorption values compared to just straightforward air plasma exposure. Also it permits air plasma treatment to be carried out at higher power levels without causing surface damage. Crosslinking of the polymer surface in this manner helps to retard the effects of oxidative degradation and formation of mobile low molecular weight species commonly associated with air plasma treatment. Similar improvements in hydrophilicity were found for other combinations of crosslinking gases (e.g N₂, He, Ne, Xe and Kr) and oxidising gases (e.g. O₂, CO₂ and H₂O).

Wettability measurements and XPS data confirm that plasma penetration extends throughout several layers of the porous polypropylene substrate. Once again, argon plasma pre-treatment significantly improves the hydrophilic stability of the surface. Optical microscopy revealed that air plasma exposure alone causes fibre agglomeration attributable to thermal damage. Whereas argon plasma treatment helps to raise the melting temperature of the fibre surfaces via crosslinking, thereby improving their structural integrity. The ability to incorporate hydrophilic groups throughout porous polymer structures is of potential commercial interest for applications such as diapers, filters, solid phase organic synthesis, and catalyst supports.

The wettability and stability towards hydrophobic recovery of plasma oxidised polymer surfaces can be significantly improved by using an argon plasma pre-treatment to cross-link the surface. This stabilises the surface against thermal degradation and the formation of low molecular weight oxidised species.

The wettability and stability of dimethyl sulphate plasma polymer films deposited onto polypropylene surfaces can be significantly improved by the use of an argon plasma crosslinking pre-treatment. The latter stabilises the polyolefin surface against thermal degradation and the formation of poorly adhered low molecular weight oligomeric species.

The two-step sequence of plasma treatments gives rise to stable wettable polymer surfaces. This entails crosslinking the surface first, followed by the deposition of hydrophilic species. The contact angle, XPS and FTIR measurements all indicate that these surfaces are stable towards hydrophobic recovery and in the case of porous substrates, both the exterior and interior interstitial surfaces can be modified by this method to yield high capacities for water absorption.

By adopting the pre-treatment step (i) comprising plasma crosslinking, the polymer substrate is stabilised prior to plasma polymerisation. Extension of these examples of the invention to porous polymer films is shown to produce a significant rise in total water absorption capacity and thus the modification steps of this invention provide significant advantages and improvements in the provision of material with super absorption characteristics. Although the invention relates to the particular advantages to be gained with super absorption materials it should be appreciated that the method and steps thereof as herein described can be of use with other forms of materials and therefore can be used as required to give required advantages.

## Claims

1. A method of applying a conditioning effect to a material substrate, said conditioning effect comprising exposing the substrate to at least two treatment steps of: (i) cross-linking of the material of either or both the exterior and internal surfaces of the substrate; and (ii) plasma modification or plasma deposition of/onto the cross-linked material.

2. A method according to claim, 1 wherein the precursor gas used in the generation of the plasma is a noble, inert or nitrogenous gas.

3. A method according to any preceding claims wherein the coating material is modified in the form of a hydrophilic layer in the first step with the plasma treatment in the second step acting to oxidise or nitrogenate the material.

4. A method according to claim 3 wherein the precursor gas or liquid used in the plasma treatment step are oxygen or nitrogen containing chemical compounds.

5. A method according to any of the preceding claims wherein an oxidation method is used in the form of ozonolysis.

6. A method according to claim 1 wherein the precursor gas or liquid used for the plasma treatment in step 2 (ii) contains fluoride.

7. A method according to claim 1 wherein the plasma used is a non-equilibrium plasma generated by a radio frequency, microwaves and/or direct current.

8. A method according to any of the preceding claims wherein the plasma power applied during the first step is in the range of 0.01 watt to 500 watts.

9. A method according to any of the preceding claims wherein the plasma power applied during the second step is in the range of 0.01 watt to 500 watts.

10. A method according to any of the preceding claims wherein the plasma power applied during either or both of the first and second steps is pulsed.

11. A method according to any of the preceding claims wherein the precursor gas or liquid introduced during either or both the first and second steps is pulsed.

12. A method according to any of the preceding claims wherein the substrate is defined as any article capable of supporting a coating applied thereto.

13. A method according to claim 12 wherein the substrate is a porous article with an exterior surface, a bulk matrix and pores extending from the exterior surface into the bulk matrix, said bulk matrix exterior and interstitial surfaces, at least in part, polymeric or oligomeric.

14. A method according to claim 13 wherein the bulk matrix is a polyolefin.

15. A method according to Claim 14 wherein the bulk matrix has a void volume ranging from 0.01% to 99%.

16. A method according to any of the preceding claims wherein step (i) is controlled such that the effect of said step is controlled to be applied to a limited depth of the material below the external surface.

17. A method according to any of the preceding claims wherein in step (ii) the effect of said step is controlled to be applied to a limited depth into the material below the external surface of the substrate.

18. A method according to any of the preceding claims wherein the plasma used in either or both steps (i) and (ii) is selectively applied to localised areas across the substrate surface and/or below the substrate surface.

19. A method according to any of the preceding claims wherein the material is an absorbent, hydrophobic polymer which is heated by step (i) to be cross linked by a noble gas plasma to improve its ability to retain liquid and render it super-absorbent.

20. A method according to claim 19 wherein the material is modified by a subsequent nitrogenating plasma as step (ii) to render said cross linked polymer compatible with amine functionalities to form a super-absorbent polymer capable of retaining large quantities of amine containing aqueous solutions.

21. A method according to any of the preceding claims wherein the substrate is a superabsorbent material.

22. A substrate having a modified surface, said surface modified by the method as set out in any of claims 1-21.

## Patentansprüche

1. Verfahren zum Anwenden eines Konditionierungseffekts auf ein Materialsubstrat, wobei der genannte Konditionierungseffekt das Unterziehen des Substrats wenigstens den folgenden beiden Behandlungsschritten beinhaltet: (i) Vernetzen des Materials der Außen- und/oder der Innenfläche des Substrats; und (ii) Plasmamodifizierung oder Plasmaablagerung des/auf das vernetzte(n) Material(s).

2. Verfahren nach Anspruch 1, bei dem das bei der Erzeugung des Plasmas verwendete Vorläufergas ein Edel-, Inert- oder Stickstoffgas ist.

3. Verfahren nach einem der vorherigen Ansprüche, bei dem das Beschichtungsmaterial in der Form einer hydrophilen Schicht im ersten Schritt modifiziert wird, wobei durch die Plasmabehandlung im zweiten Schritt das Material oxidiert oder nitrogenisiert wird.

4. Verfahren nach Anspruch 3, bei dem das/die im Plasmabehandlungsschritt eingesetzte Vorläufergas oder -flüssigkeit sauerstoff- oder stickstoffhaltige chemische Verbindungen sind.

5. Verfahren nach einem der vorherigen Ansprüche, bei dem ein Oxidationsverfahren in der Form von Ozonolyse angewendet wird.

6. Verfahren nach Anspruch 1, bei dem das/die im Plasmabehandlung in Schritt 2 (ii) eingesetzte Vorläufergas Fluorid enthält.

7. Verfahren nach Anspruch 1, bei dem das verwendete Plasma ein Nichtgleichgewichtsplasma ist, das durch eine Funkfrequenz, durch Mikrowellen und/oder durch Gleichstrom erzeugt wird.

8. Verfahren nach einem der vorherigen Ansprüche, bei dem die im ersten Schritt angewendete Plasmaleistung im Bereich von 0,01 Watt bis 500 Watt liegt.

9. Verfahren nach einem der vorherigen Ansprüche, bei dem die im zweiten Schritt angewendete Plasmaleistung im Bereich von 0,01 Watt bis 500 Watt liegt.

10. Verfahren nach einem der vorherigen Ansprüche, bei dem die im ersten und/oder zweiten Schritt angewendete Plasmaleistung pulsiert wird.

11. Verfahren nach einem der vorherigen Ansprüche, bei dem das/die im ersten und/oder im zweiten Schritt eingeleitete Vorläufergas oder -flüssigkeit pulsiert wird.

12. Verfahren nach einem der vorherigen Ansprüche, bei dem das Substrat als ein Artikel definiert ist, der eine darauf aufgebrachte Beschichtung tragen kann.

13. Verfahren nach Anspruch 12, bei dem das Substrat ein poröser Artikel mit einer Außenfläche ist, wobei eine Massenmatrix und Poren von der Außenfläche in die Massenmatrix verlaufen, wobei die Außen- und Einlagerungsflächen der genannten Massenmatrix, wenigstens teilweise, polymer oder oligomer sind.

14. Verfahren nach Anspruch 13, bei dem die Massenmatrix ein Polyolefin ist.

15. Verfahren nach Anspruch 14, bei dem die Massenmatrix ein Hohlraumvolumen im Bereich von 0,01 bis 99% hat.

16. Verfahren nach einem der vorherigen Ansprüche, bei dem Schritt (i) so gesteuert wird, dass der Effekt des genannten Schrittes so reguliert wird, dass die Applikation bis auf eine begrenzte Tiefe des Materials unter der Außenfläche erfolgt.

17. Verfahren nach einem der vorherigen Ansprüche, bei dem in Schritt (ii) der Effekt des genannten Schrittes so reguliert wird, dass die Applikation bis auf eine begrenzte Tiefe des Materials unter der Außenfläche des Substrats erfolgt.

18. Verfahren nach einem der vorherigen Ansprüche, bei dem das in Schritt (i) und/oder (ii) verwendete Plasma selektiv auf lokalisierte Bereiche über der Substratoberfläche und/oder unter der Substratoberfläche appliziert wird.

19. Verfahren nach einem der vorherigen Ansprüche, bei dem das Material ein absorptionsfähiges, hydrophobes Polymer ist, das in Schritt (i) zum Vernetzen durch ein Edelgasplasma erhitzt wird, um seine Flüssigkeitsretentionsfähigkeit zu verbessern und um es superabsorbierend zu machen.

20. Verfahren nach Anspruch 19, bei dem das Material durch ein nachfolgendes Nitrogenisierplasma als Schritt (ii) modifiziert wird, um das genannte vernetzte Polymer mit Aminfunktionalitäten kompatibel zu machen, um ein superabsorbierendes Polymer zu bilden, das große Mengen an aminhaltigen wässrigen Lösungen zu halten.

21. Verfahren nach einem der vorherigen Ansprüche, bei dem das Substrat ein superabsorbierendes Material ist.

22. Substrat mit einer modifizierten Oberfläche, wobei die genannte Oberfläche mit einem Verfahren gemäß einem der Ansprüche 1 bis 21 modifiziert ist.

## Revendications

1. Procédé d'application d'un effet de conditionnement à un substrat de matériau, ledit effet de conditionnement comprenant l'exposition du substrat à au moins deux étapes de traitement, à savoir : (i) la réticulation du matériau de l'une ou des deux surfaces du substrat, notamment sa surface externe et sa surface interne ; et (ii) la modification du plasma ou le dépôt du plasma du matériau réticulé ou sur le matériau réticulé.

2. Procédé, selon la revendication 1, dans lequel le gaz précurseur utilisé dans la génération du plasma est un gaz noble, inerte ou azoté.

3. Procédé, selon l'une quelconque des revendications précédentes, dans lequel le matériau de revêtement est modifié pour prendre la forme d'une couche hydrophile lors de la première étape, alors que le traitement au plasma lors de la deuxième étape agit de façon à oxyder ou à azoter le matériau.

4. Procédé, selon la revendication 3, dans lequel le gaz ou le liquide précurseur utilisé lors de l'étape de traitement au plasma sont des composés chimiques contenant de l'oxygène ou de l'azote.

5. Procédé, selon l'une quelconque des revendications précédentes, dans lequel on utilise un procédé d'oxydation sous la forme d'une ozonolyse.

6. Procédé, selon la revendication 1, dans lequel le gaz ou le liquide précurseur utilisé pour le traitement au plasma lors de l'étape de l'étape 2 (ii) contient du fluorure.

7. Procédé, selon la revendication 1, dans lequel le plasma utilisé est un plasma hors-équilibre qui est généré par une radiofréquence, des hyperfréquences et/ou un courant continu.

8. Procédé, selon l'une quelconque des revendications précédentes, dans lequel la puissance de plasma appliquée pendant la première étape se trouve dans la gamme de 0,01 watt à 500 watts.

9. Procédé, selon l'une quelconque des revendications précédentes, dans lequel la puissance de plasma appliquée pendant la deuxième étape se trouve dans la gamme de 0,01 watt à 500 watts.

10. Procédé, selon l'une quelconque des revendications précédentes, dans lequel la puissance de plasma appliquée pendant la première étape ou la deuxième étape, ou pendant les deux étapes, est pulsée.

11. Procédé, selon l'une quelconque des revendications précédentes, dans lequel le gaz ou le liquide précurseur introduit pendant la première étape ou la deuxième étape, ou pendant les deux étapes, est pulsé.

12. Procédé, selon l'une quelconque des revendications précédentes, dans lequel le substrat est défini comme étant n'importe quel article capable de supporter un revêtement qui lui est appliqué.

13. Procédé, selon la revendication 12, dans lequel le substrat est un article poreux ayant une surface externe, une matrice en masse et des pores qui s'étendent depuis la surface externe jusque dans la matrice en masse, alors que la face externe de la matrice en masse et les surfaces interstitielles sont polymères ou oligomériques, au moins en partie.

14. Procédé, selon la revendication 13, dans lequel la matrice en masse est une polyoléfine.

15. Procédé, selon la revendication 14, dans lequel la matrice en masse possède un volume de vide qui se trouve dans la gamme de 0,01 % à 99%.

16. Procédé, selon l'une quelconque des revendications précédentes, dans lequel l'étape (i) est contrôlée de sorte que l'effet de ladite étape soit contrôlé de façon à être appliqué sur une profondeur limitée du matériau situé sous la surface externe.

17. Procédé, selon l'une quelconque des revendications précédentes, dans lequel dans l'étape (ii) l'effet de ladite étape est contrôlé de façon à être appliqué sur une profondeur limitée dans le matériau situé sous la surface externe du substrat.

18. Procédé, selon l'une quelconque des revendications précédentes, dans lequel le plasma utilisé dans l'étape (i) ou dans l'étape (ii), ou dans les deux, est sélectivement appliqué sur des zones localisées sur l'ensemble de la surface du substrat et/ou en dessous de la surface du substrat.

19. Procédé, selon l'une quelconque des revendications précédentes, dans lequel le matériau est un polymère absorbant hydrophobe qui est chauffé lors de l'étape (i) afin d'être réticulé par un plasma à gaz noble dans le but d'améliorer son aptitude à retenir du liquide et à le rendre super-absorbant.

20. Procédé, selon la revendication 19, dans lequel le matériau est modifié par un plasma d'azotisation ultérieur en tant qu'étape (ii) afin de rendre ledit polymère réticulé compatible avec les fonctionnalités amines dans le but de former un polymère super-absorbant capable de retenir de grandes quantités de solutions aqueuses contenant des amines.

21. Procédé, selon l'une quelconque des revendications précédentes, dans lequel le substrat est un matériau superabsorbant.

22. Substrat avec une surface modifiée, ladite surface étant modifiée par le procédé tel qu'il est énoncé dans l'une quelconque des revendications 1 à 21.
